Europäisches Patentamt

European Patent Office

Office européen des brevets

(19)

(11) **EP 0 495 346 B1**

(12) **FASCICULE DE BREVET EUROPEEN**

(45) Date de publication et mention
de la délivrance du brevet:
**15.05.1996 Bulletin 1996/20**

(51) Int Cl.[6]: **C12Q 1/42**, G01N 27/26,
B01D 57/02
// C12Q1/00

(21) Numéro de dépôt: **91870218.4**

(22) Date de dépôt: **23.12.1991**

(54) **Procédé de séparation, d'identification et de quantification d'isoenzymes et d'isoformes de la phosphatase alcaline**

Verfahren zur Abtrennung, Identifizierung und Quantifizierung von Isoenzymen und Isoformen der alkalischen Phosphatase

Procedure for separation, identification, and quantification of isoenzymes and isoforms of the alkaline phosphatase

(84) Etats contractants désignés:
**BE CH DE ES FR GB IT LI NL SE**

(30) Priorité: **15.01.1991 BE 9100026**

(43) Date de publication de la demande:
**22.07.1992 Bulletin 1992/30**

(73) Titulaire: **ANALIS S.A.**
**B-5000 Namur (BE)**

(72) Inventeur: **Chevigne, Roland**
**B-5100 Wepion (BE)**

(74) Mandataire: **Van Malderen, Michel et al**
**Office van Malderen**
**85/043 Boulevard de la Sauvenière**
**4000 Liège (BE)**

(56) Documents cités:
**US-A- 4 030 995**

- **CLINICAL CHEMISTRY, vol. 28, no. 10, octobre 1982, Winston, NC (US); D.W. MOSS et al., pp. 2007-2016/**
- **BIOCHEMICAL MEDICINE, vol. 9, no. 3, mars 1974, New York, NY (US); L. FISHMAN, pp. 309-315/**

**Description**

Objet de l'invention

La présente invention concerne essentiellement un procédé électrophorétique permettant de séparer et d'identifier les isoenzymes et les isoformes de la phosphatase alcaline présentes dans un liquide physiologique ou un extrait de tissu.

Elle concerne également un procédé densitométrique permettant de quantifier les isoenzymes et les isoformes mises en évidence par le procédé électrophorétique de l'invention.

L'invention s'étend également aux préparations utilisées pour la mise en oeuvre du procédé et aux produits de celui-ci.

**Arrière-plan technologique**

Le terme de phosphatase alcaline (EC3.1.3.1), en abrégé PAL, regroupe un ensemble d'enzymes catalysant, à pH basique, l'hydrolyse d'esters phosphoriques.

La PAL est présente au niveau des membranes cellulaires et se trouve dans la plupart des tissus de l'organisme. En fonction de leur localisation, on parlera des isoenzymes intestinale, rénale, osseuse, hépatique, placentaire et intestin foetal. Les PAL circulent également dans le sérum sanguin.

Dans l'organisme humain, quatre gènes différents contrôlent l'expression de la PAL: un gène tissulaire non spécifique (codant pour les isoenzymes osseuse, hépatique et rénale, notamment), un gène intestinal, un gène placentaire et un gène intestin foetal, chacun de ces gènes codant pour une isoenzyme correspondante.

Chacune de ces isoenzymes peut subir une ou plusieurs modifications post-translationnelles altérant ses propriétés physico-chimiques mais conservant ses propriétés biologiques et devenir une isoforme.

On connaît actuellement les isoformes suivantes: isoenzyme liée à une immunoglobuline, isoenzyme tétramère, isoenzyme ayant une teneur anormale en acide sialique, isoenzyme liée à un débris de membrane cellulaire (appelé macro-moléculaire), isoenzyme liée à une lipoprotéine, par exemple à la LpX.

Le nombre et la quantité des différentes isoenzymes et isoformes de la phosphatase alcaline varient très fortement en fonction d'une part de l'âge et d'autre part de l'état de santé des individus.

Les isoenzymes osseuses et hépatiques ont été particulièrement étudiées, car toute pathologie primaire ou secondaire affectant les systèmes osseux et/ou hépato-biliaire est susceptible d'occasionner une augmentation ou une diminution selon le cas des isoenzymes osseuses et/ou hépatiques dans le sérum sanguin et de s'accompagner de l'apparition d'une autre isoenzyme ou d'une ou plusieurs isoformes.

On sait que le taux d'isoenzymes osseuses sériques décroît de l'enfance vers l'âge adulte et présente des pointes au moment des poussées de croissance. Un arrêt de croissance chez l'enfant se marque par une diminution anormale du taux d'isoenzymes osseuses. Les poussées de croissance s'accompagnent de la présence, dans le sérum sanguin, d'une seconde fraction osseuse qui apparaît sous forme de tétramères.

Cette fraction est absente du sérum de l'adulte normal, mais on l'y découvre lorsqu'un processus multiplicatif pathologique est présent au niveau des os (maladie de Paget, métastases osseuses...).

Une activité ostéoclastique sera caractérisée par la présence d'isoenzyme osseuse à teneur moindre en acide sialique.

L'isoenzyme hépatique reste normalement à un taux constant tout au long de la vie d'un individu en bonne santé.

Les maladies dégénératives du foie comme la fibrose, les maladies occasionnant une infiltration hépatique et résultant en une destruction progressive des hépatocytes, résultent en une diminution, puis en une disparition de l'isoenzyme hépatique.

Une cholostase intra ou extrahépatique (hépatite, cirrhose, métastases hépatiques, compression hépatique occasionnée par une tumeur de voisinage par exemple) peut occasionner par contre une augmentation de l'isoenzyme hépatique accompagnée de l'apparition de l'isoforme dénommée fraction macromoléculaire, hépato-biliaire ou alpha 1, (liée à un débris de membrane) ainsi que d'autres isoformes, telles que des fractions hépatiques liées à différentes lipoprotéines.

En l'absence d'une inflammation aiguë, la présence d'une isoenzyme hépatique ayant une teneur en acide sialique diminuée est un signe de présence tumorale.

Une isoenzyme hépatique ayant une teneur anormale en acide sialique se rencontre dans l'hyperphosphatasémie transitoire infantile (infection virale des voies respiratoires ou digestives chez l'enfant).

L'identification d'autres types d'isoenzymes de la phosphatase alcaline permet également de déceler des troubles pathologiques.

On sait ainsi que la présence d'isoenzymes placentaires en dehors de la grossesse est le signe d'une pathologie tumorale à évolution rapide, que des isoenzymes et isoformes intestinales en quantités élevées peuvent être la marque d'une maladie systémique et que l'isoenzyme intestin foetal se rencontre en cas de tumeur primitive du foie (hépatome).

L'étude des phosphatases alcalines peut donc apporter une aide substantielle au diagnostic mais aussi à la surveillance des patients pour suivre l'évolution d'une maladie ou objectiver l'effet d'un traitement.

**Résumé de l'état de la technique**

Les différentes isoenzymes sont habituellement séparées par électrophorèse sur gel d'agarose ou de polyacrylamide ou sur membranes d'acétate de cellulose.

Selon les techniques utilisées, certaines isoenzymes ou isoformes ne sont pas révélées ou ne peuvent être identifiées sans ambiguïté. La sensibilité n'est pas toujours suffisante non plus, ce qui empêche une détection précoce des pathologies.

L'influence de l'utilisation de détergents non-ioniques sur les résultats de différents types d'électrophorèse est décrite dans la littérature. FISHMAN L., dans l'article intitulé "Acrylamide disc gel electrophoresis of alkaline phosphate of human tissues, serum and ascites fluid using triton X-100 in the sample and the gel matrix" et publié dans Biochemical Medicine, Vol. 9, n° 3, mars 1974, pages 309-315, a montré que l'utilisation du détergent non-ionique Triton X100 dans divers échantillons et dans un gel d'acrylamide permet de faire pénétrer dans ce gel des isoenzymes de haut poids moléculaire qui, en l'absence de ce détergent, restent au point d'application et apparaissent comme une zone diffuse.

Ceci est confirmé dans l'article de MOSS et al. intitulé "Alkaline phosphatase isoenzymes" et publié dans CLINICAL CHEMISTRY, vol. 28, n° 10, octobre 1982, pages 2007-2016, Winston-Salem, Washington, US.

On a aussi proposé dans le brevet US-A-4 030 995 d'utiliser différents détergents non-ioniques de poids moléculaire compris entre 600 et 2000 tels que Triton, Brij ou Tween pour l'électrophorèse de phosphatases alcalines sur un gel d'agarose ou une membrane de cellulose. Les isoenzymes pénètrent dans le support avec amélioration de la sensibilité mais sans influence sur la qualité de la séparation.

Les détergents anioniques, quant à eux, apportent une charge négative aux différentes isoformes, permettant de les étaler sur toute la longueur de la migration. Leur utilisation présente cependant des inconvénients: ils augmentent le courant (avec le risque de rupture des gels) et par conséquent l'effet Joule (dénaturation possible des enzymes); ces détergents sont en outre souvent dénaturants au niveau des protéines (donc des enzymes), ils peuvent même être des "anti-enzymes", c'est-à-dire causer une inhibition de l'activité phophatasique. Enfin, ils peuvent conférer une charge trop grande pour une isoforme donnée, la faire migrer trop fortement lors de l'électrophorèse et même la faire sortir du gel.

Lorsqu'on utilise un détergent cationique seul, la fraction macromoléculaire dont la charge est modifiée migre dans la direction opposée à celle qu'elle adopte en électrophorèse de protéines sériques, ce qui rend difficile l'interprétation.

Pour toutes ces raisons, les détergents anioniques ou cationiques ne sont habituellement guère utilisés dans les techniques électrophorétiques pour l'identification des phosphatases alcalines.

Jusqu'à présent, une séparation correcte et une identification sûre des isoenzymes osseuse et hépatique notamment n'ont pu être obtenues qu'en recourant à des techniques complémentaires impliquant une double analyse.

Ainsi, il est connu de pratiquer des électrophorèses sur membranes d'acétate de cellulose en faisant migrer sur le même support non seulement l'échantillon à analyser mais encore un échantillon thermodénaturé 10 minutes à 56°C.

Il est encore connu de traiter des échantillons à la neuraminidase, qui ôte les résidus d'acide sialique de phosphatases alcalines à des vitesses différentes selon l'origine (hépatique ou osseuse) de l'isoenzyme, ce qui assure une meilleure séparation des bandes électrophorétiques correspondant aux isoenzymes hépatique ou osseuse. D'après les techniques connues, même l'analyse densitométrique ne permet pas d'observer d'une manière nette deux fractions séparées sans recourir à un test complémentaire. Il est à noter que la neuraminidase n'agit pas sur la fraction intestinale, constituée d'asialoglycoprotéines.

Les techniques à double analyse sont malheureusement lentes et coûteuses.

La durée des analyses selon les procédés connus incluant l'utilisation d'un détergent est encore augmentée par le fait que le détergent est généralement ajouté au gel après le coulage et le refroidissement de celui-ci: avant l'électrophorèse proprement dite, il est nécessaire de placer le gel dans un tampon dit d'équilibration pendant 30 minutes. De tels tampons n'ont qu'une durée de conservation limitée (60 jours) et doivent donc être renouvelés lorsqu'on pratique des analyses à longueur d'année.

## Buts visés par l'invention

L'invention a pour but de mettre au point une technique électrophorétique qui permette une séparation correcte des isoenzymes osseuse et hépatique de la phosphatase alcaline ainsi qu'une identification sans ambiguïté de toute autre isoenzyme ou isoforme, tout en ayant une très grande sensibilité.

Elle a également pour but de permettre une telle séparation qui puisse être réalisée sans recourir à une double analyse d'échantillons.

Elle a encore pour but de fournir un procédé électrophorétique permettant de quantifier directement les isoenzymes osseuse et hépatique de la phosphatase alcaline dans du liquide physiologique ou des extraits de tissus.

Un but particulièrement intéressant est de fournir des trousses d'électrophorèse prêtes à l'emploi, de longue conservation et qui permettent d'obtenir une bonne coloration des échantillons tout en pouvant être utilisées au cours d'une procédure plus simple et dont la durée est considérablement diminuée par rapport aux procédures à appliquer dans les techniques connues.

La Demanderesse a constaté avec surprise que l'utilisation conjointe de détergent non-ionique et de détergent anionique dans un gel d'agarose utilisé en électrophorèse permettait d'atteindre les buts ci-dessus.

## Eléments caractéristiques de l'invention

Selon l'invention, on fournit un procédé électrophorétique pour séparer et identifier les isoenzymes et les isoformes de la phosphatase alcaline dans un échantillon de liquide physiologique ou d'extrait de tissu dans lequel on applique sur le milieu de support un tampon, au moins un détergent non-ionique et au moins un détergent anionique. Les détergents sont de préférence en solution dans le tampon.

De préférence, chaque détergent est présent dans le tampon en une quantité de 0,1 à 10 g/l.

Avantageusement, la proportion entre le détergent non-ionique et le détergent anionique est 2:1.

De préférence, le pH du tampon est compris entre 8 et 11. Dans une forme d'exécution préférée, le pH est de 9,45.

Le tampon utilisé est de préférence du Tris(hydroxyméthyl) aminométhane contenant de l'acide borique.

Le détergent anionique peut être choisi parmi les acides biliaires et les sels biliaires et consister dans ce cas de préférence en désoxycholate de sodium ou en taurocholate de sodium.

Alternativement, le détergent anionique peut consister en un composé de formule

$$R_1 - XNa$$

où

R$_1$ représente une chaîne alkyle linéaire ou ramifiée de 6 à 12 atomes de carbone et

X représente un groupe choisi parmi les groupes sulfate, sulfonate et carboxylate.

On peut également utiliser comme détergent anionique dans le procédé de l'invention un dérivé d'acide aminé dont la fonction amine porte un substituant N-alkyle ou N-alkoyle de 6 à 12 atomes de carbone.

Le milieu de support pour l'électrophorèse peut être une membrane d'acétate de cellulose, un gel de polyacrylamide ou un gel d'agarose.

Dans ce dernier cas, les détergents peuvent être ajoutés soit à la solution tamponnée d'agarose avant le coulage des gels, soit, en mélange dans un tampon d'équilibration, au gel déjà coulé.

Le détergent non-ionique est choisi dans le groupe comprenant les dérivés de type Triton® (alkylphenylpolyethyleneglycol), Brij® (polyoxyethyleneether) et Tween® (polyoxyethylenesorbitan), le lubrol, l'éthylphénylpolyoxyéthylène glycol et le N-oxyde de diméthyl N-N dodécylamine.

Le procédé de l'invention peut comprendre en outre une étape de quantification des isoenzymes et des isoformes de la phosphatase alcaline séparées et identifiées par ce procédé. Cette quantification peut être réalisée par analyse densitométrique aux environs de 600 nm après incubation du gel pendant 15 minutes dans une solution contenant du 5-bromo-4-chloro-3-indolyl phosphate et du bleu de tétrazolium nitré.

L'invention a également pour objet des trousses pour l'électrophorèse des isoenzymes de la phosphatase alcaline comprenant un gel d'agarose et un agent détergent consistant en au moins un détergent non-ionique et au moins un détergent anionique. De préférence, le gel d'agarose de ces trousses est fabriqué en y introduisant les détergents en solution dans un tampon, avant la solidification du gel contenant de l'agarose.

Les détergents sont choisis parmi ceux mentionnés ci-dessus.

Un autre objet de l'invention est un gel d'agarose contenant ces détergents.

Avantageusement, on fabrique les trousses de l'invention en introduisant, avant solidification de l'agarose, outre les détergents, de la chlorhexidine. Cette substance est un bon agent conservateur; un objet de l'invention est son utilisation à cette fin dans des réactifs de laboratoire.

Un autre objet de l'invention est l'utilisation des trousses de l'invention pour l'identification et la quantification des isoenzymes et des isoformes dans des échantillons de liquide physiologique et des extraits de tissus.

## Exemples d'exécution de l'invention

L'invention est illustrée par les exemples de préparation de gels électrophorétiques repris ci-dessous (exemples 1 à 4) et par un exemple de mise en oeuvre (exemple 5).

### Exemples 1 à 4 (préparation)

Une solution standard d'agarose est préparée de la manière suivante: à 1 l d'eau distillée sont ajoutés successivement: 0,38 mole de Tris(hydroxyméthyl) aminométhane, 0,062 mole d'acide borique, environ 0,02 mole d'hydroxyde de sodium pour obtenir un pH de 9,45. Après dissolution complète, 10 g d'agarose ainsi que des adjuvants connus permettant d'obtenir les caractéristiques de gel souhaitées (texture, rétention d'eau,...), sont ajoutés sous forte agitation. Le mélange est porté à 95°C. Après dissolution complète, il est refroidi à 55°C.

### Exemple 1

1 g d'éthylphénylpolyoxyéthylène glycol renfermant 10 résidus éthylène et 0,75 g de N-lauroylsarcosinate de sodium ainsi que 200 mg de chlorhexidine sont ajoutés à la solution standard. Après dissolution complète, l'agitation est arrêtée et on procède au coulage des gels d'agarose (200 à 250 gels de 10 x 7,5 cm) sur un support adéquat.

### Exemple 2

1 g d'éthylphénylpolyoxyéthylène glycol renfermant 10 résidus éthylène et 0,9 g de N-lauroyl-N-méthyltaurinate de sodium ainsi que 200 mg de chlorhexidine sont ajoutés à la solution standard. Après dissolution complète, l'agitation est arrêtée et on procède au coulage des gels d'agarose (200 à 250 gels de 10 x 7,5 cm) sur un support adéquat.

### Exemple 3

1 g de polyoxyéthylène lauryl éther, PEG 9-10, (PM moyen 582) renfermant 10 résidus éthylène et 0,75 g de N-lauroylsarcosinate de sodium ainsi que 200 mg de chlorhexidine sont ajoutés à la solution standard. Après dissolution complète, l'agitation est arrêtée et on procède au coulage des gels d'agarose (200 à 250 gels de 10 x 7,5 cm) sur un support adéquat.

### Exemple 4

1 g de N-oxyde de diméthyl N-N dodécylamine renfermant 10 résidus éthylène et 0,75 g de N-lauroylsarcosinate de sodium ainsi que 200 mg de chlorhexidine sont ajoutés à la solution standard. Après dissolution complète, l'agitation est arrêtée et on procède au coulage des gels d'agarose (200 à 250 gels de 10 x 7,5 cm) sur un support adéquat.

Il est à noter que l'utilisation de la chlorhexidine, (que l'on utilise habituellement de manière courante pour la désinfection des mains pendant des opérations chirurgicales, ou en tant que désinfectant actif dans des affections buccales, par exemple) en tant qu'agent conservateur dans un gel d'agarose est particulièrement avantageuse: les résultats obtenus sont tout aussi bons qu'en utilisant classiquement l'azide de sodium, lequel risque de former des composés explosifs au contact d'objets métalliques, par exemple dans les canalisations d'eau.

La Demanderesse a d'ailleurs utilisé la chlorhexidine avec succès à cette fin pour des gels d'agarose ou d'agar destinés à d'autres types d'analyses électrophorétiques que celles des isoenzymes de la phosphatase alcaline. L'utilisation de chlorhexidine comme agent préservatif empêchant le développement de bactéries, de moisissures, levures et algues dans des réactifs de laboratoire les plus divers s'est, à maintes reprises, révélée efficace.

### Exemple 5 (mise en oeuvre)

Un gel obtenu selon une des préparations décrites en exemples 1, 2, 3, 4 est déposé sur la table, buvardé pour enlever l'excès de tampon et des échantillons sont appliqués selon un procédé courant en électrophorèse. Le gel est alors soumis à une électrophorèse de 150 V pendant 25 minutes (valeurs pour un gel de 10 x 7,5 cm) en utilisant de préférence comme tampon d'électrophorèse un tampon identique à celui utilisé pour la préparation de la solution standard d'agarose. Après l'électrophorèse, le gel est incubé à 45°C pendant 15 minutes avec une solution de 10 ml d'un tampon 2-amino-2-méthylpropanol à pH 10,6 contenant du substrat 5-bromo-4-chloro-3-indolyl phosphate et du bleu de tétrazolium nitré. Le gel est alors rincé avec de l'eau distillée et/ou une solution d'acide acétique à 5%, puis séché. Après rinçage, le gel peut également être buvardé, compressé une ou plusieurs fois, puis séché.

Il est à noter par ailleurs que le bleu de tétrazolium nitré amplifie la coloration obtenue grâce à la transformation du substrat en un composé violet. Un quart d'heure d'incubation dans le tampon est tout à fait suffisant pour obtenir un excellent résultat.

Les gels présentent, après électrophorèse, une série bandes de coloration violette correspondant aux différentes isoformes ou isoenzymes contenues dans les échantillons et peuvent dans la plupart des cas être identifiées à l'oeil nu.

Des résultats expérimentaux obtenus avec un grand nombre d'échantillons de différentes origines ont permis d'identifier d'une manière fiable et sans recourir à une analyse complémentaire, les isoenzymes et isoformes suivantes: isoenzymes liées à des lipoprotéines, isoenzyme liée à la LpX, fraction X présente dans l'hyperphosphatasémie transitoire infantile, la fraction macromoléculaire, la fraction intestin foetal, des fractions tétramérisées (telles que la seconde fraction osseuse, la deuxième et la troisième fractions placentaires, la fraction variant intestinal), la fraction hépatique, la fraction osseuse, la fraction intestinale adulte, et les fractions hépatique, osseuse, intestinale placentaire, chacune de ces fractions se présentant sous une forme liée à une immunoglobuline.

L'analyse densitométrique aux environs de 600 nm permet de déterminer avec précision les quantités des différentes fractions présentes dans un échantillon donné. Si de telles analyses sont effectuées sur un même patient au cours du temps, elles permettent un suivi de l'évolution d'une maladie, ou un diagnostic précis.

Bien que les exemples ci-dessus illustrent des formes d'exécution particulièrement avantageuses de l'invention, ils n'en limitent nullement la portée.

Un mélange de détergent non-ionique et de détergent anionique dans un tampon peut également être utilisé dans des techniques d'électrophorèse dans lesquelles le support est constitué par de l'acétate de cellulose par exemple

Au cas où on dispose par exemple d'un gel Beckman Paragon SPE ne contenant pas un tel tampon à deux détergents tel que décrit plus haut, on ne sortira pas du cadre de l'invention en laissant incuber un tel gel classique dans un tel tampon: les résultats obtenus bien que la procédure soit plus longue, sont tout à fait similaires; une telle manière de procéder constitue une alternative qui ne sort pas du cadre de l'invention.

## Revendications

1. Procédé électrophorétique pour séparer et identifier les isoenzymes et les isoformes de la phosphatase alcaline dans un échantillon de liquide physiologique ou d'extrait de tissu dans lequel on applique sur un milieu de support un tampon et au moins un détergent non-ionique, caractérisé en ce qu'en plus dudit détergent non-ionique, on applique au moins un détergent anionique.

2. Procédé selon la revendication 1, caractérisé en ce que les détergents sont en solution dans le tampon.

3. Procédé selon la revendication 2, caractérisé en ce que chaque détergent est présent dans le tampon en une quantité de 0,1 à 10 g/l de tampon.

4. Procédé selon l'une quelconque des revendications précédentes, caractérisé en ce que la proportion entre le détergent non-ionique et le détergent anionique est 2:1.

5. Procédé selon l'une quelconque des revendications précédentes, caractérisé en ce que le pH du tampon est compris entre 8 et 11.

6. Procédé selon l'une quelconque des revendications précédentes, caractérisé en ce que le tampon utilisé est du Tris(hydroxyméthyl) aminométhane contenant de l'acide borique.

7. Procédé selon l'une quelconque des revendications précédentes, caractérisé en ce que le détergent anionique est choisi parmi les acides biliaires et les sels biliaires.

8. Procédé selon la revendication 7, caractérisé en ce que le détergent est un sel biliaire, de préférence le désoxycholate de sodium ou le taurocholate de sodium.

9. Procédé selon l'une quelconque des revendications 1 à 6, caractérisé en ce que le détergent anionique consiste en un composé de formule

$$R_1 \, X \, Na$$

où $R_1$ représente une chaîne alkyle linéaire ou ramifiée de 6 à 12 atomes de carbone,
et X représente un groupe choisi parmi les groupes sulfate, sulfonate et carboxylate.

10. Procédé selon l'une quelconque des revendications 1 à 6, caractérisé en ce que le détergent anionique est un dérivé d'acide aminé dont la fonction amine porte un substituant N-alkyle ou N-alkoyle de 6 à 12 atomes de carbone.

11. Procédé selon l'une quelconque des revendications précédentes, caractérisé en ce que le milieu de support est une membrane d'acétate de cellulose.

12. Procédé selon l'une quelconque des revendications 1 à 10, caractérisé en ce que le milieu de support est un gel de polyacrylamide.

13. Procédé selon l'une quelconque des revendications 1 à 10, caractérisé en ce que le milieu de support est un gel d'agarose.

14. Procédé selon la revendication 13, caractérisé en ce que les détergents sont ajoutés à une solution tamponnée d'agarose avant le coulage des gels.

15. Procédé selon la revendication 13, caractérisé en ce que les détergents sont ajoutés en mélange dans un tampon d'équilibration au gel d'agarose déjà coulé.

16. Procédé suivant l'une quelconque des revendications précédentes, caractérisé en ce que le détergent non-ionique est choisi dans le groupe comprenant les dérivés de type alkylphénylpolyéthylèneglycol, polyoxyéthylène éther, polyoxyéthylène sorbitan, le lubrol, l'éthylphénylpolyoxyéthylène glycol et le N-oxyde de diméthyl-N-N-dodécylamine.

17. Procédé selon l'une quelconque des revendications précédentes, caractérisé en ce qu'il comporte en outre une étape de quantification des isoenzymes et des isoformes de la phosphatase alcaline séparés et identifiés.

18. Procédé selon la revendication 17, caractérisé en ce qu'on incube le gel après électrophorèse pendant 15 minutes avec un substrat contenant du S-bromo-4-chloroindolyl phosphate et du bleu de tétrazolium nitré et en ce qu'on procède à une analyse densitométrique aux environs de 600 nm des bandes colorées obtenues, ce qui permet une quantification.

19. Gel d'agarose pour l'électrophorèse des isoenzymes de la phosphatase alcaline, caractérisé en ce qu'il contient en tant qu'agent détergent au moins un détergent non-ionique et au moins un détergent anionique.

20. Gel selon la revendication 19, caractérisé en ce que le ou les détergents anioniques sont choisis parmi les détergents d'une quelconque des revendications 7 à 10 et en ce que le ou les détergents non-ioniques sont choisis parmi les détergents de la revendication 16.

21. Gel selon la revendication 19 ou 20 caractérisé en ce qu'il comprend également de la chlorhexidine.

22. Procédé de fabrication du gel selon l'une quelconque des revendications 19 à 21 pour l'électrophorèse des isoenzymes de la phosphatase alcaline, caractérisé en ce qu'on prépare une solution standard d'agarose dans un tampon, qu'on la chauffe, qu'on la laisse refroidir, et qu'on y ajoute au moins un détergent non-ionique et au moins un détergent anionique avant solidification.

23. Procédé suivant la revendication 22, caractérisé en ce qu'on ajoute également, avant solidification, de la chlorhexidine.

24. Trousse pour l'électrophorèse des isoenzymes de la phosphatase alcaline comprenant un gel d'agarose selon l'une quelconque des revendications 19 à 21.

25. utilisation d'une trousse suivant la revendication 24 pour l'identification et la quantification des isoenzymes et isoformes de la phosphatase alcaline dans des échantillons de liquide physiologique et des extraits de tissu.

**Patentansprüche**

1. Elektrophoretisches verfahren, um die Isoenzyme und die Isoforme der alkalischen Phosphatase in einer Probe einer physiologischen Flüssigkeit oder eines Gewebeextrakts abzutrennen und zu identifizieren, bei dem ein Puffer und mindestens ein nicht-ionisches Detergens auf ein Trägermedium aufgebracht wird, dadurch gekennzeichnet, daß zusätzlich zu dem nicht-ionischen Detergens mindestens ein anionisches Detergens aufgebracht wird.

2. Verfahren gemäß Anspruch 1, dadurch gekennzeichnet, daß die Detergenzien in dem Puffer in Lösung sind.

3. Verfahren gemäß Anspruch 2, dadurch gekennzeichnet, daß jedes Detergens in dem Puffer in einer Menge von 0,1 bis 10 g/l Puffer enthalten ist.

4. Verfahren gemäß irgendeinem der vorhergehenden Ansprüche, dadurch gekennzeichnet, daß das Verhältnis zwischen dem nicht-ionischen Detergens und dem anionischen Detergens 2:1 ist.

5. Verfahren gemäß irgendeinem der vorhergehenden Ansprüche, dadurch gekennzeichnet, daß der pH des Puffers zwischen 8 und 11 liegt.

6. Verfahren gemäß irgendeinem der vorhergehenden Ansprüche, dadurch gekennzeichnet, daß der verwendete Puffer Tris(hydroxymethyl)aminomethan ist, das Borsäure enthält.

7. Verfahren gemäß irgendeinem der vorhergehenden Ansprüche, dadurch gekennzeichnet, daß das anionische Detergens unter den Gallensäuren und den Gallensalzen gewählt wird.

8. Verfahren gemäß Anspruch 7, dadurch gekennzeichnet, daß das Detergens ein Gallensalz, vorzugsweise Natriumdesoxycholat oder Natriumtaurocholat ist.

9. Verfahren gemäß irgendeinem der Ansprüche 1 bis 6, dadurch gekennzeichnet, daß das anionische Detergens aus einer verbindung mit der Formel

$$R_1 \ X \ Na$$

besteht,

wo $R_1$ eine lineare oder verzweigte Alkylkette mit 6 bis 12 Kohlenstoffatomen repräsentiert,
und X eine Gruppe repräsentiert, die unter den Gruppen Sulfat, Sulfonat und Carboxylat ausgewählt wird.

10. Verfahren gemäß irgendeinem der Ansprüche 1 bis 6, dadurch gekennzeichnet, daß das anionische Detergens ein Aminosäurederivat ist, dessen Amingruppe einen N-Alkyl- oder N-Alkoyl-Substituenten mit 6 bis 12 Kohlenstoffatomen trägt.

11. Verfahren gemäß irgendeinem der vorhergehenden Ansprüche, dadurch gekennzeichnet, daß das Trägermedium eine Zelluloseacetat-Membran ist.

12. Verfahren gemäß irgendeinem der Ansprüche 1 bis 10, dadurch gekennzeichnet, daß das Trägermedium ein Polyacrylamid-Gel ist.

13. Verfahren gemäß irgendeinem der Ansprüche 1 bis 10, dadurch gekennzeichnet, daß das Trägermedium ein Agarose-Gel ist.

14. Verfahren gemäß Anspruch 13, dadurch gekennzeichnet, daß die Detergenzien vor dem Gießen der Gele zu einer gepufferten Agaroselösung hinzugegeben werden.

15. Verfahren gemäß Anspruch 13, dadurch gekennzeichnet, daß die Detergenzien in einem Äquilibrationspuffer zu dem bereits gegossenen Agarose-Gel hinzugegeben werden.

16. Verfahren gemäß irgendeinem der vorhergehenden Ansprüche, dadurch gekennzeichnet, daß das nicht-ionische Detergens in der Gruppe gewählt wird, die die Derivate vom Typ Alkylphenylpolyäthylenglykol, Polyoxyäthylenäther, Polyoxyäthylensorbitan, Lubrol, Äthylphenylpolyoxyäthylenglykol und N-Oxid von Dimethyl-N-N-dodecylamin aufweist.

17. Verfahren gemäß irgendeinem der vorhergehenden Ansprüche, dadurch gekennzeichnet, daß es außerdem einen Schritt zur Quantifizierung der abgetrennten und identifizierten Isoenzyme und Isoforme der alkalischen Phosphatase umfaßt.

18. Verfahren gemäß Anspruch 17, dadurch gekennzeichnet, daß das Gel nach der Elektrophorese während 15 Minuten mit einem Substrat inkubiert wird, das S-Brom-4-chlor-indolylphosphat und Tetrazolnitritblau enthält, und daß bei ungefähr 600 nm eine densitometrische Analyse der erhaltenen gefärbten Streifen gemacht wird, was eine Quantifizierung ermöglicht.

19. Agarose-Gel für die Elektrophorese der Isoenzyme der alkalischen Phosphatase, dadurch gekennzeichnet, daß es als Detergens mindestens ein nicht-ionisches Detergens und mindestens ein anionisches Detergens enthält.

20. Gel gemäß Anspruch 19, dadurch gekennzeichnet, daß das oder die anionischen Detergenzien unter den Detergenzien von irgendeinem der Ansprüche 7 bis 10 gewählt werden, und daß das oder die nicht-ionischen Detergenzien unter den Detergenzien des Anspruchs 16 gewählt werden.

21. Gel gemäß Anspruch 19 oder 20, dadurch gekennzeichnet, daß es außerdem Chlorhexidin aufweist.

22. Verfahren zur Herstellung des Gels gemäß irgendeinem der Ansprüche 19 bis 21 für die Elektrophorese der Isoenzyme der alkalischen Phosphatase, dadurch gekennzeichnet, daß eine Standard-Agaroselösung in einem Puffer angesetzt wird, die Lösung erhitzt wird, und die Lösung abgekühlt wird, und daß vor der Verfestigung mindestens ein nicht-ionisches Detergens und mindestens ein anionisches Detergens hinzugegeben wird.

23. Verfahren gemäß Anspruch 22, dadurch gekennzeichnet, daß vor der Verfestigung außerdem Chlorhexidin hinzugegeben wird.

24. Ausrüstung für die Elektrophorese der Isoenzyme der alkalischen Phosphatase, wobei diese Ausrüstung ein Agarose-Gel gemäß irgendeinem der Ansprüche 19 bis 21 aufweist.

25. Verwendung einer Ausrüstung gemäß Anspruch 24 zur Identifizierung und Quantifizierung der Isoenzyme und Isoforme der alkalischen Phosphatase bei Proben von physiologischer Flüssigkeit und bei Gewebeextrakten.

## Claims

1. Electrophoresis process to separate and identify isoenzymes and isoforms from alcaline phosphatase in a physiological liquid or tissue extract sample, wherein on a backing medium a buffer and at least one non ionic detergent are applied, characterized in that besides said non ionic detergent at least one anionic detergent is applied.

2. Process according to claim 1, characterized in that the detergents are in solution in the buffer.

3. Process according to claim 2, characterized in that each detergent is present in the buffer in a quantity of 0.1 to 10 g/l buffer.

4. Process according to any of preceding claims, characterized in that the ratio of the non ionic detergent to the anionic detergent is 2:1.

5. Process according to any of preceding claims, characterized in that the buffer pH is comprised between 8 and 11.

6. Process according to any of preceding claims, characterized in that the buffer used is tris(hydroxymethyl) aminomethane containing boric acid.

7. Process according to any of preceding claims, characterized in that the anionic detergent is selected amongst biliary acids and biliary salts.

8. Process according to claim 7, characterized in that the detergent is a biliary salt, preferably sodium desoxycholate or sodium taurocholate.

9. Process according to any of claims 1 to 6, characterized in that the anionic detergent consists in a compound of formula :

$$R_1 \, X \, Na$$

wherein :

$R_1$ represents a linear or branched alkyl chain of 6 to 12 atoms of carbon, and
X represents a group selected amongst sulfate, sulfonate and carboxylate groups.

10. Process according to any of claims 1 to 6, characterized in that the anionic detergent is an amino acid derivative the amine function of which bears an N-alkyl or N-alkoyl substituent of 6 to 12 atoms of carbon.

**11.** Process according to any of preceding claims, characterized in that the backing medium is a membrane of cellulose acetate.

**12.** Process according to any of claims 1 to 10, characterized in that the backing medium is a polyacrylamide gel.

**13.** Process according to any of claims 1 to 10, characterized in that the backing medium is an agarose gel.

**14.** Process according to claim 13, characterized in that the detergents are added to an agarose buffered solution before casting of the gels.

**15.** Process according to claim 13, characterized in that the detergents are added by mixing in an equilibration buffer to the already cast agarose gel.

**16.** Process according to any of preceding claims, characterized in that the non ionic detergent is selected in the group comprising the alkylphenylpolyethyleneglycol-, polyoxyethylene ether- and polyoxyethylene sorbitan-type derivates, lubrol, ethylphenylpolyoxyethylene glycol and dimethyl-N-N-dodecylamine-N-oxide.

**17.** Process according to any of preceding claims, characterized in that it comprises also a quantification step for the separated and identified isoenzymes and isoforms of alcaline phosphatase.

**18.** Process according to claim 17, characterized in that the gel after an electrophoresis in incubated for 15 minutes with a substrate containing 5-bromo-4-chloro-indolyl phosphate and nitrated methylene blue and in that a densitometric analysis is carried out at around 600 nm from the coloured stripes obtained, which allows to make a quantification.

**19.** Agarose gel for electrophoresis of isoenzymes of alcaline phosphatase, characterized in that it contains as a detergent agent at least one non ionic detergent and at least one anionic detergent.

**20.** Gel according to claim 19, characterized in that the anionic detergent(s) is (are) selected amongst the detergents of any of claims 7 to 10 and in that the non ionic detergent(s) is (are) selected amongst the detergents of claim 16.

**21.** Gel according to claim 19 or 20, characterized in that it also comprises chlorhexidine.

**22.** Process for manufacturing gel according to any of claims 19 to 21 for electrophoresis of isoenzymes of alcaline phosphatase, characterized in that a standard agarose solution is prepared in a buffer, heated and allowed to cool, and in that at least one non ionic detergent and at least one anionic detergent are added to it before solidification.

**23.** Process according to claim 22, characterized in that chlorhexidine is also added before solidification.

**24.** Kit for electrophoresis of isoenzymes of alcaline phosphatase, comprising an agarose gel according to any of claims 19 to 21.

**25.** Use of a kit according to claim 24 to identify and quantify isoenzymes and isoforms of alcaline phosphatase in samples of physiological liquid and tissue extracts.